(19) Europäisches Patentamt
European Patent Office
Office européen des brevets

(11) Veröffentlichungsnummer: **0 174 572 B1**

(12) # EUROPÄISCHE PATENTSCHRIFT

(45) Veröffentlichungstag der Patentschrift: **06.03.91**

(51) Int. Cl.⁵: **C07C 233/64**, G01N 27/40

(21) Anmeldenummer: **85110977.7**

(22) Anmeldetag: **30.08.85**

Die Akte enthält technische Angaben, die nach dem Eingang der Anmeldung eingereicht wurden und die nicht in dieser Patentschrift enthalten sind.

(54) **Neue Cyclohexan-1,2-dicarbonsäurediamide, ionenselektive Membranen sowie Testvorrichtungen, die diese enthalten.**

(30) Priorität: **13.09.84 CH 4424/84**

(43) Veröffentlichungstag der Anmeldung:
**19.03.86 Patentblatt 86/12**

(45) Bekanntmachung des Hinweises auf die Patenterteilung:
**06.03.91 Patentblatt 91/10**

(84) Benannte Vertragsstaaten:
**AT BE CH DE FR GB IT LI LU NL SE**

(56) Entgegenhaltungen:

**CHEMICAL ABSTRACTS, Band 96, Nr. 7, 15. Februar 1982, Seite 271, Spalte 2, Zusammenfassungsnr. 48437g, Columbus, Ohio, US; A.F. ZHUKOV et al.: "Improved lithium ion-selective electrode based on a lipophilic diamide as neutral carrier"**

**CHEMICAL ABSTRACTS, Band 97, Nr 3, 19. Juli 1982, Seite 654, Spalte 2, Zusammenfassungsnr. 23195j, Columbus, Ohio, US; D. ERNE et al.: "Lipophilic diamides as ionophores for alkali and alkaline earth metal cations"**

(73) Patentinhaber: **Willi Möller AG**
**Gubelstrasse 37**
**CH-8050 Zürich(CH)**

(72) Erfinder: **Wilhelm, Simon, Prof. Dr.**
**Rütistrasse 32**
**CH-8702 Zollikon(CH)**

(74) Vertreter: **Blum, Rudolf Emil Ernst et al**
**c/o E. Blum & Co Patentanwälte Vorderberg 11**
**CH-8044 Zürich(CH)**

**EP 0 174 572 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 95, Nr. 2, 13. Juli 1981, Seite 409, Spalte 1, Zusammenfassungsnr. 13335z, Columbus, Ohio, US; W. BUSSMANN et al.: "Enantiomer-selectivity for phenylethylammonium ion of membranes based on a chiral macrocyclic polyether"

ANALYTICAL CHEMISTRY, Band 53, Nr. 13, November 1981, Seiten 1970-1974, Spalte 1, Zeilen 43-47, Washington DC, US; P. ANKER et al.: "Neutral carrier based ion-selective electrode for the determination of total calcium in blood serum"

CHIMIA, Band 38, Nr. 12, Dezember 1984, Seiten 440-442, Zürich, CH; E. METZGER et al.: "Lipophilic neutral carriers for lithium selective liquid membrane electrodes"

HELV.CHIM.ACTA, 65, 538-545 (1982)

## Beschreibung

### HINTERGRUND DER ERFINDUNG

Die vorliegende Erfindung betrifft neue Cyclohexan-1,2-dicarbonsäurediamide. Diese neuen Cyclohexan-1,2-dicarbonsäurediamide besitzen eine hohe Selektivität für. Lithiumionen, im Vergleich zu anderen Alkalimetallionen. Sie können als ionenselektive Komponente in einer ionenselektiven Membran zur Bestimmung der Konzentration von Lithiumionen und in Testvorrichtungen, beispielsweise Teststreifen, zur Feststellung von Lithiumionen in flüssigen Medien eingesetzt werden.

### BESCHREIBUNG DES STANDES DER TECHNIK

Verschiedene Dicarbonsäurediamide, die mit Kationen lipophile Komplexe bilden und die als Komponenten von ionenselektiven Membranen zur Bestimmung der Konzentration der fraglichen Kationen eingesetzt werden können, sind bereits in der Literatur beschrieben. Unter diesen bisher beschriebenen Dicarbonsäurediamiden befinden sich auch solche, die eine gewisse Selektivität für Lithiumionen im Vergleich zu anderen Alkalimetallionen besitzen.

Die Konzentrationen der wichtigsten Kationen, die in biologischen Flüssigkeiten anzutreffen sind, nämlich von $Na^+$, $K^+$, $Ca^{2+}$ und Wasserstoffionen können in klinischen Laboratorien leicht bestimmt werden, indem man Elektroden einsetzt, die mit einer entsprechenden ionenselektiven Membran ausgestattet sind. Im Gegensatz dazu wird die Testung auf Lithiumionen immer noch nach der Methode der Flammenphotometrie oder der Atomabsorptions-Spektrometrie durchgeführt. Die Feststellung der Konzentration von Lithiumionen im Blutserum ist dann sehr wichtig, wenn Patienten, die an manisch-depressiven Psychosen leiden, einer Therapie mit Lithiumionen unterworfen werden oder wenn an Patienten Lithiumionen prophylaktisch verabreicht werden, um das Auftreten von manisch-depressiven Psychosen zu verhindern. Es sei in diesem Zusammenhang auf die Veröffentlichungen von A. Amidsen und M. Schou in Münch. Med. Wochenschr., 117(1975) 1417 und A. Amidsen, Dan. Med. Bull., 22(1975) 277 hingewiesen.

Bekanntlich liegen im Blutserum wesentlich höhere Konzentrationen an Natriumionen vor als an Lithiumionen und deshalb ist für eine ionenselektive Membran, die in einer Elektrode verwendet wird, um die Aktivität der Lithiumionen im Blutserum zu bestimmen, ein hoher Selektivitätskoeffizient für Lithiumionen, im Vergleich zu anderen Alkalimetallionen und insbesondere im Vergleich zu Natriumionen, unbedingt nötig. Eine ionenselektive Komponente einer ionenselektiven Membran zur Bestimmung von Lithiumionen in Körperflüssigkeiten muss daher hohen Anforderungen bezüglich ihrer $Li^+/Na^+$-Selektivität genügen. Ausserdem müssen Elektroden, die mit derartigen lithiumselektiven Membranen ausgestattet sind, eine adequate Stabilität des Elektrodenpotentials aufweisen.

Es wurden bereits viele neue Diamide von Dicarbonsäuren hergestellt, um entsprechende komplexbildende Mittel zu synthetisieren, welche die gewünschte hohe Selektivität gegenüber Lithiumionen im Vergleich zu anderen Alkalimetallionen und Erdalkalimetallionen aufweisen, um solche Dicarbonsäurediamide zu finden, die dementsprechend als ionenselektive Komponente in entsprechenden für Lithiumionen selektiven Membranen eingesetzt werden.

Elektroden, welche eine ionenselektive Membran aufweisen und welche Dicarbonsäurediamide enthalten, die eine Selektivität für Lithiumionen gegenüber Natriumionen aufweisen, sind in der Veröffentlichung von A.F. Zhukov, D. Erne, D. Amman, M. Güggi, E. Pretsch und W. Simon in Analytice Chimica Acta, 131-(1981) auf den Seiten 117 - 122 beschrieben.

In dieser Veröffentlichung ist auch diejenige Verbindung genannt, die von den bisher bekannten Carbonsäurediamiden in den für medizinische Zwecke nicht besonders geeigneten Membranen mit o-Nitrophenyloctyläther als Weichmacher, jedoch ohne Tetraphenylboratzugabe, den höchsten Selektivitätskoeffizienten für Lithiumionen im Vergleich zu anderen Alkalimetallionen aufweist, nämlich einen Selektivitätskoeffizienten, der grösser als 100 ist. Diese Verbindung ist ein Cyclohexancarbonsäurediamid, welches die folgende Formel A

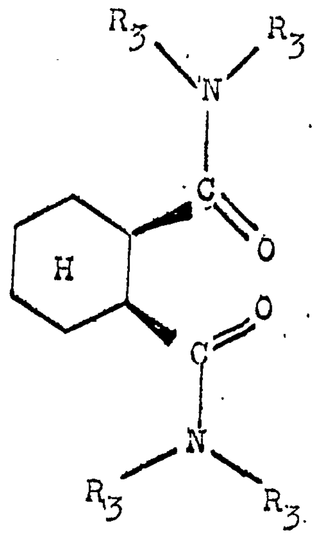

A

aufweist, wobei in dieser Formel die beiden Gruppierungen der Formel

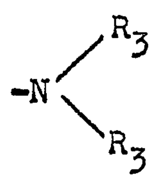

die folgende Struktur besitzen:

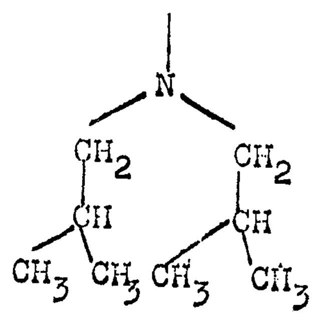

Diese Verbindung der Formel A ist also das N,N,N',N'-Tetraisobutylcyclohexan-cis-1,2-dicarbonsäurediamid.

Die Synthese dieser Verbindung der Formel A ist in der Veröffentlichung von D. Erne, D. Ammann, A.F. Zhukov, F. Behm, E. Pretsch und W. Simon, in Helv. Chim. Acta, 65 (1982), Seiten 538-545, beschrieben. Wie aus Seite 544, Zeilen 4 bis 11 dieser Veröffentlichung ersichtlich ist, erfolgt die Herstellung dieses Cyclohexan-cis-1,2-dicarbonsäure-diamides dadurch, dass man das Cyclohexan-dicarbonsäureanhydrid mit dem entsprechenden Amin umsetzt. Diese Umsetzung erfolgt in zwei Reaktionsstufen, und zwar wird zunächst ein Moläquivalent des Cyclohexan-dicarbonsäureanhydrides mit einem Moläquivalent des Amines bei Zimmertemperatur während mehrerer Stunden umgesetzt, wobei man als Zwischenprodukt das entsprechende Cyclohexan-dicarbonsäuremonoamid erhält. Dieses wird dann mit Dicyclohexylcarbodiimid und mit der Aminkomponente des Dicarbonsäurediamides zu dem Cyclohexan-dicarbonsäurediamid umgesetzt. Diese Umsetzung liefert die cis-Form des Cyclohexan-1,2-dicarbonsäure-diamides.

In dieser Veröffentlichung ist ferner die Herstellung der Transform eines Cyclohexandicarbonsäurediamides erläutert, nämlich der Verbindung der Formel 13 auf Seite 540 dieser Veröffentlichung. Wie man aus den Erläuterungen auf Seite 542 dieser Veröffentlichung sieht, erfolgt die Herstellung des dort beschriebenen trans-Cyclohexan-1,2-dicarbonsäure-diamides durch Umsetzung von einem Moläquivalent des Dicarbonsäure-dichlorides mit vier Moläquivalenten Amin in Toluol bei Zimmertemperatur während

mehrerer Stunden.

## BESCHREIBUNG DER ERFINDUNG

Es wurde nun in völlig unerwarteter Weise gefunden, dass zwei Verbindungen, die bezüglich ihrer chemischen Struktur dem Cyclohexancarbonsäurediamid der Formel A sehr ähnlich aufgebaut sind, eine $Li^+/Na^+$-Selektivität aufweisen, die etwa dem Zehnfachen der Selektivität der Verbindung der Formel A entsprechen, wenn diese neuen Dicarbonsäurediamide als ionenselektive Komponente in solchen ionenselektiven Membranen eingesetzt werden, die für die Anwendung auf dem medinischen Sektor besonders geeignet sind. Derartige Membranen enthalten einen lipophilen Weichmacher und vorzugsweise als weitere Komponente Kalium-tetrakis(p-chlorphenyl) borat oder Tetraphenylborat.

Ein Gegenstand der vorliegenden Erfindung sind daher neue Cyclohexan-cis-1,2-dicarbonsäurediamide, die dadurch gekennzeichnet sind, dass sie die folgende Formel I

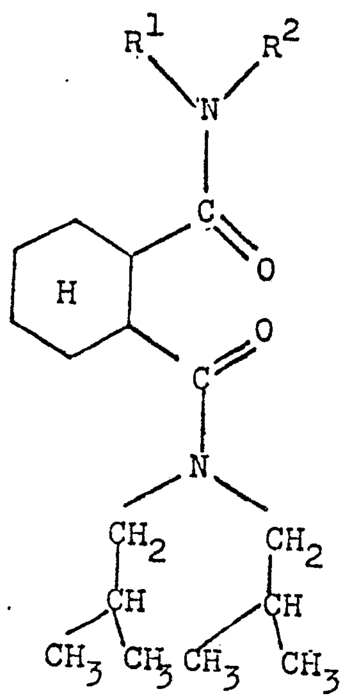

I

aufweisen, worin die Gruppierung der Formel

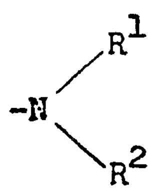

entweder die Formel

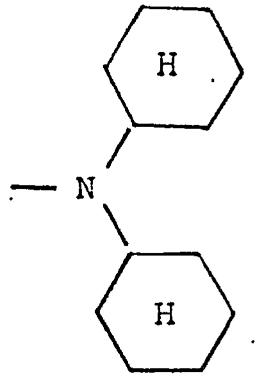

oder die Formel

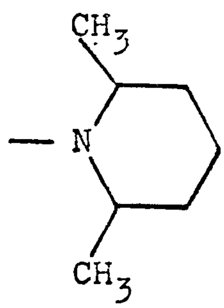

aufweist.

Ein weiterer Gegenstand der vorliegenden Erfindung ist eine ionenselektive Membran zur Bestimmung der Konzentration von Lithiumionen, die dadurch gekennzeichnet ist, dass sie als ionenselektive Komponente eine erfindungsgemässe Verbindung der Formel I enthält.

Des woiteren betrifft die vorliegende Erfindung Testvorrichtungen, die zum raschen Test auf Lithiumionen in flüssigen Medien eingesetzt wer-den können und die als Komponente eine erfindungsgemässe Verbindung der Formel I enthalten.

Eine der beiden erfindungsgemässen Verbindungen der Formel I ist das N,N-DicyclohexanN',N'-diisobutyl-cis-cyclohexan-1,2-dicarboxamid der Formel Ia

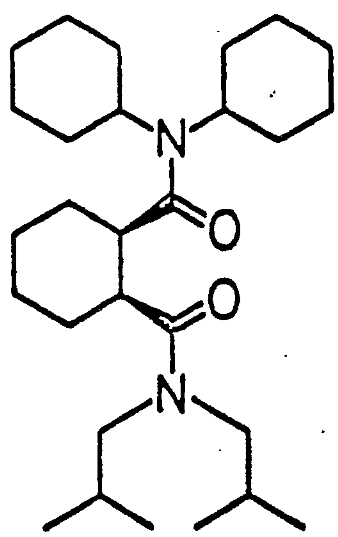

Ia

Die erfindungsgemässen ionenselektiven Membranen, welche als ionenselektive Komponente ein neues Cyclohexan-cis-1,2-dicarbonsäurediamid der Formel I enthalten, weisen vorzugsweise als Matrix für die ionenselektive Komponente Poly(vinylchlorid) auf. Speziell bevorzugt sind solche erfindungsgemässen

ionenzelektiven Membranen, die ferner einen Weichmacher enthalten, vorzugsweise einen lipophilen Weichmacher, wie o-Nitrophenyl-octyläther oder Bis(1-butyl-pentyl)adipat. Von den erfindungsgemässen ionenselektiven Membranen, die als ionenselektive Komponente eine Verbindung der Formel I enthalten, sind diejenigen speziell bevorzugt, deren Matrix aus Poly(vinylchlorid) aufgebaut ist und die ausserdem einen Weichmacher enthalten, vorzugsweise einen der oben genannten Weichmacher und ferner als weitere Komponente mindestens eine Substanz, wie Tetraphenylborat oder Kalium-tetrakis (p-chlorphenyl)borat.

Erfindungsgemässe Membranen, welche die erfindungsgemässen Verbindungen der Formel I enthalten und Membranen, welche die Verbindungen zu Vergleichszwecken enthalten, wurde in Zellanordnungen getestet und die elektromotorische Kraft (abgekürzt als EMK) wurde bestimmt.

Die EMK-Untersuchungen der Membranen, welche die erfindungsgemässen Verbindungen der Formel I enthalten und der Membranen zu Vergleichszwecken, wurden unter Verwendung der folgenden Anordnung durchgeführt:

Hg; $Hg_2Cl_2$, KCl (ges.) | 3 M KCl | Probenlösung || ionenselektive Membran || Elektrodenfüllungslösung, AgCl; Ag.

Die Referenzelektrode war eine gesättigte Kalomelelektrode mit zwei seriellen Diffusionspotentialübergängen und einem freien Flüssigkeitsübergang von l µl/h. Wenn die Bestimmungen nach der Methode der getrennten Lösung (separate solution method) durchgeführt wurden, dann wurde als Elektrodenfüllungslösung eine 0,001 molare LiCl Lö sung verwendet, während dann, wenn die Messung nach der Methode des konstanten Störionenhintergrundes (fixed interference method) durchgeführt wurde, als Elektrodenfüllung eine 0,001 molare Lithiumchloridlösung eingesetzt wurde, die auch die störenden Kationen in solchen Konzentrationen enthielten, die für extrazellulare Verhältnisse typisch sind.

Die ionenselektiven Membranen wurden nach dem Verfahren hergestellt, das von Anker, P.; Wieland, E.; Ammann,D.; Dohner, R.; Asper, R. und Simon, W. in Anal. Chem. 1981 , 53, 1970 beschrieben ist, indem man eine Verbindung der Formel I als ionenselektive Komponente einsetzte oder eine Verbindung zu Vergleichszwecken als ionenselektive Komponente einsetzte und ferner Polyvinylchlorid als Grundmaterial verwendete und gegebenenfalls ausserdem einen der weiter oben genannten Weichmacher und der weiter vorne genannten weiteren Komponenten verwendete.

Die Membranen wurden in Philips Elektrodenkörpern IS 560 (N.V. Philips, Eindhoven, NL) montiert und die Elektroden über Nacht in etwa 2 ml der Elektrodenfüllungslösung konditioniert, ehe sie verwendet wurden.

Mit entsprechenden ionenselektiven Membranen, welche die erfindungsgemässen Verbindungen oder die Verbindungen zu Vergleichszwecken enthielten, wurde der Wert

$$\log K^{Pot}_{LiNa}$$

bestimmt.

Für die bereits bekannte Verbindung der Formel A ergab sich dabei ein Wert für

$$\log K^{Pot}_{LiNa}$$

von -1,0. Im Gegensatz dazu besitzen beide erfindungsgemässen Verbindungen einen Wert für von -2,3.

$$\log K^{Pot}_{LiNa}$$

Es wurde ferner zu Vergleichszwecken eine ebenfalls neue Verbindung der folgenden Formel B

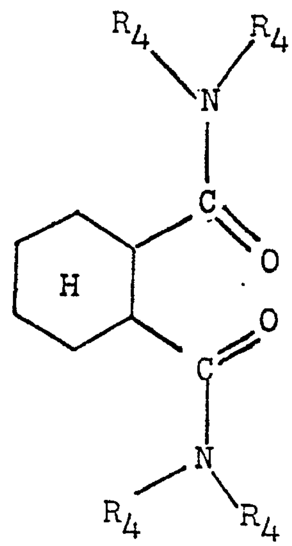

B

synthetisiert, in welcher beide Reste der Formel

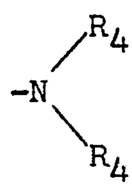

Gruppierungen der Formel

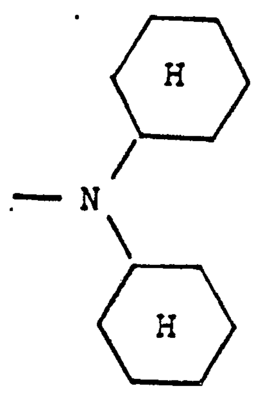

sind.

Ueberraschenderweise hatten diese Verbindungen nur eine geringfügig höhere Selektivität für Lithiumionen gegenüber Natriumionen als die bekannten Verbindungen der Formel A, nämlich der entsprechende Wert für

$$\log K^{Pot}_{LiNa}$$

betrug -1,3.

Ein weiterer Gegenstand der vorliegenden Erfindung ist eine Testvorrichtung zur Bestimmung von Lithiumionen, welche als Komponenten ein Cyclohexan-cis-1,2-dicarbonsäurediamid der Formel I enthält.

Derartige Testvorrichtungen, wie zum Beispiel Bänder oder Streifen, enthalten vorzugsweise eine Polymermatrix, z.B. aus Polyvinylchlorid, und ausserdem einen Indikator, der es erlaubt, eine Aenderung des pH-Wertes festzustellen. Dieser Indikator kann beispielsweise einer der üblichen pH-Indikatoren sein, die eine Aenderung des pH-Wertes durch einen Farbwechsel anzeigen. Wenn eine solche Testvorrichtung, die als ionenselektive Komponente ein Cyclohexan-cis-1,2-dicarbonsäureamid der Formel I enthält, mit einem flüssigen Medium in Kontakt gebracht wird, welches Lithiumionen enthält, dann bildet sich in dieser Testvorrichtung ein Komplex zwischen den Lithiumionen und den Verbindungen der Formel I aus und durch diese Komplexbildungsreaktion wird ausserdem der pH-Wert in der Testvorrichtung geändert. Diese Veränderung des pH-Wertes ist durch ein Ansprechen des pH-Indikators feststellbar.

Die erfindungsgemässen Testvorrichtungen, beispielsweise Teststreifen, ermöglichen es, Lithiumionen in flüssigen Medien, wie zum Beispiel Körperflüssigkeiten, rasch und einfach festzustellen.

Die Herstellung der neuen Verbindungen der Formel I erfolgt nach den Arbeitsverfahren, die in der weiter vorne genannten Veröffentlichung von D. Erne et al. in Helv. Chim. Acta, 65 (1982), Seiten 538-545, bereits beschrieben sind.

Die Herstellung der cis-Form des Cyclohexan-dicarbonsäure-diamides der Formel I erfolgte nach dem folgenden Reaktionsschema:

Durch Umsetzung des Cyclohexandicarbonsäureanhydrides der Formel II mit einem entsprechenden Amin der Formel III

$$NHR_1R_2 \qquad III$$

wird als Zwischenprodukt das entsprechende Cyclohexan-dicarbonsäure-monoamid der Formel IV hergestellt. Diese Synthese des Zwischenproduktes sei anhand des folgenden Reaktionsschemas erläutert:

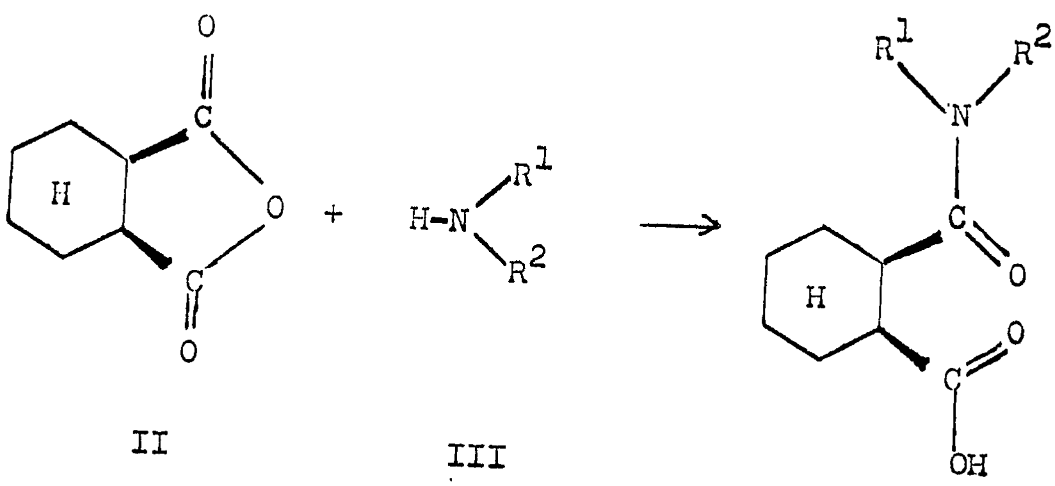

II III IV

Das Zwischenprodukt der Formel IV wird durch Umkristallisieren gereinigt und dann in Anwesenheit von Dicyclohexylcarbodiimid mit dem Amin der Formel VI

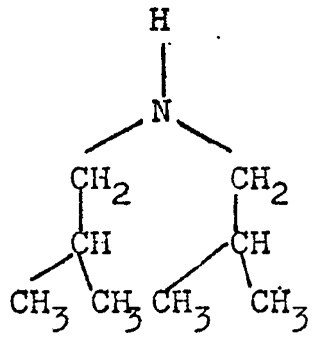

VI

zu dem gewünschten Endprodukt der Formel I umgesetzt.

Diese Syntheseverfahren seien anhand der folgenden Beispiele noch näher erläutert.

Beispiel 1

A) Herstellung des Cyclohexandicarbonsäuremono amid-Zwischenproduktes

Als Amin der Formel III wurde das Amin der Formel

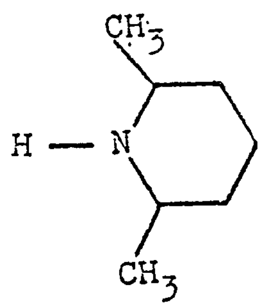

verwendet.

1 Moläquivalent des cis-Cyclohexandicarbonsäureanhydrides der Formel II, welches ein Molekulargewicht von 154,17 aufweist, wird mit einem Moläquivalent des Amines der Formel III in Toluol versetzt. Man kocht während fünf Stunden unter Rückfluss.

Anschliessend wird das Toluol eingedampft und der Rückstand aus einer Mischung von 9 Volumenteilen Essigsäureäthylester und 1 Volumenteil Chloroform umkristallisiert. Das erhaltene Cyclohexandicarbonsäuremonoamid besitzt ein Molekulargewicht von 267,37.

B) Herstellung des entsprechenden Cyclohexan dicarbonsäurediamides der Formel I

1 Moläquivalent des als Zwischenprodukt erhaltenen Cyclohexandicarbonsäuremonoamides der Formel IV wird mit 1,15 Moläquivalenten des Amines der Formel VI und 1,06 Moläquivalenten Dicyclohexylcarbodiimid in Methylenchlorid versetzt. Die Mischung wird etwa 30 Stunden lang bei Zimmertemperatur gerührt. Anschliessend wird der gebildete Harnstoff abfiltriert und das Methylenchlorid eingedampft, wobei man die cis-form der, entsprechenden Verbindung der Formel I als Rückstand erhält.

Dieses Rohprodukt wird zwei mal durch Flash-Chromatographie gereinigt. Das erste Mal mit einer Mischung aus 8 Volumenteilen Hexan plus 2 Volumenteile Essigsäureäthylester.

Das zweite Mal unter Verwendung einer Mischung aus 7 Volumenteilen Hexan plus 3 Volumenteile Essigsäureäthylester.

Schliesslich wird das gewonnene Produkt im Kugelrohr destilliert. Es besitzt bei einem Druck von 0,05 mm Quecksilbersäule einen Siedepunkt von 140° C.

Die Ergebnisse der Mikroanalyse waren wie folgt:

| | | | |
|---|---|---|---|
| berechnet: | C 72,97 | H 11,18 | N 7,40 |
| gefunden: | C 72,81 | H 10,82 | N 7,73 |

Molekulargewicht: 378,60.

Beispiel 2

A) Herstellung des Cyclohexandicarbonsäuremono amides der Formel IV

Bei diesem Verfahren wurde als Amin der Formel III das Amin der Formel

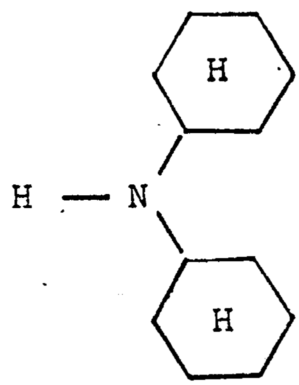

verwendet.

1 Moläquivalent des cis-Cyclohexandicarbonsäureanhydrides der Formel II,(Molgewicht 154,17) wurde mit einem Moläquivalent des Amines in Toluol versetzt. Man rührte die Mischung bei etwa 90° C während 10 Stunden und anschliessend etwa 7 Stunden lang bei Zimmertemperatur. Anschliessend wird das Toluol abgedampft und das Cyclohexandicarbonsäuremonoamid durch Umkristallisieren aus einer Mischung von 9 Volumenteilen Hexan und 1 Volumenteil Essigsäureäthylester gereinigt. Das erhaltene Cyclohexandicarbonsäuremonoamid der Formel IV wies ein Molekulargewicht von 335,49 auf.

B) Herstellung des entsprechenden Cyclohexan dicarbonsäurediamides der Formel I

1 Moläquivalent des Cyclohexandicarbonsäuremonoamides wurde mit 1,1 Moläquivalenten des Amins der Formel V und 1,1 Moläquivalenten Dicyclohexylcarbodiimid in Methylenchlorid versetzt.

Man rührte bei Zimmertemperatur 15 Stunden lang.

Anschliessend wurde der ausgefallene Harnstoff abfiltriert, aus dem Filtrat das Methylenchlorid abgedampft, wobei man als Rückstand die cis form des Cyclohexandicarbonsäurediamides als Rohprodukt erhielt.

Dieses Rohprodukt wurde in Diäthyläther aufgelöst, zwei mal mit je 0,1 normaler Chlorwasserstoffsäure, 0,1 normaler Natronlauge und Wasser ausgeschüttelt, der Aether eingeengt und es wurde eine zweimalige Flash-Chromatographie mit Chloroform durchgeführt.

Das erhaltene Endprodukt wies ohne Kristallwasser ein Molekulargewicht von 446,72 auf. Der Schmelzpunkt des 1 Molkristallwasser enthaltenden Endproduktes lag über 250° C.

Die Ergebnisse der Mikroanalyse waren wie folgt:

berechnet (mit 1 Mol Wasser): C 72,37 H 11,28 N 6,03
gefunden: C 72,45 H 11,24 N 6,36.

Beispiel 3

Anhand dieses Beispiels wird ein weiteres Verfahren zur Herstellung der Verbindung der Formel Ia beschrieben. In diesem Falle wird jedoch nicht wie in Beispiel 2 das Monohydrat der Verbindung der Formel Ia hergestellt, sondern die wasserfreie Verbindung der Formel Ia.

A) Herstellung des Cyclohexan-dicarbonsäure monoamid-Zwischenproduktes

Eine Lösung von 4,6 g (30 mMol) des cis-Hexahydrophthalsäureanhydrides (purum, Fluka AG, Buchs, Schweiz) und 5,4 g (30 mMol) des Dicyclohexylamines (puriss. p.a., Fluka AG) in 100 ml Toluol wurde 18 Stunden lang unter Rückfluss gekocht. Dann wurde das Lösungsmittel unter Vakuum entfernt und der Rückstand aus einer Mischung von 9 Teilen Hexan plus 1 Teil Essigsäureäthylester umkristallisiert. Man erhielt 6,4 g (19 mMol, 63,3 % der Theorie) an dem Zwischenprodukt.

B) Herstellung des Cyclohexan-dicarbonsäure diamides der Formel Ia

4,5 g (22 mMol) an dem Dicyolohexylcarbodiimid in 50 ml trockenem Methylenchlorid wurden bei Zimmertemperatur zu einer gerührten Lösung von 6,4 g (19 mMol) an der cis-2-N,N-Dicyclo-hexylcarbamoylcyclohexancarbonsäure, die in Schritt A hergestellt wurde und 2,9 g (22 mMol) an Diisobutylamin (purum, destilliert, Fluka AG) in 150 ml trockenen Methylenchlorid zugesetzt. Die Reaktionsmischung wurde bei Zimmertemperatur über Nacht gerührt. Der ausgefallene Dicyclohexyl-harnstoff wurde abfiltriert und das Filtrat eingedampft. Das Rohprodukt wurde durch eine dreimalige Flashchromotographie (40 kPa) auf Silicagel gereinigt, indem man Chloroform als erstes und zweites Laufmittel einsetzte und eine Mischung aus 9 Teilen Hexan plus 1 Teil Essigsäureäthylester als drittes Laufmittel verwendete. Man erhielt so 1,7 g (3,8 mMol, 20 % der Theorie) an dem reinen Produkt.

Das wasserfreie Produkt der Formel Ia, nämlich das N,N-Dicyclohexyl-N',N'-diisobutyl-cis-cyclohexan-1,2-dicarbonsäurediamid hatte einen Schmelzpunkt von 112 - 113° C und das Molekulargewicht für diese Verbindung der Summenformel $C_{28}H_{50}N_2O_2$ betrug 446,72.

In der Elementaranalyse wurden die folgenden Ergebnisse erhalten:

berechnet: C 75,28 H 11,28 N 6,27
gefunden: C 75,38 H 11,21 N 6,27.

Das Infrarotspektrum und das $^1$H-NMR waren in guter Uebereinstimmung mit der Konstitution.

Beispiel 4

Herstellung einer ionenselektiven Membran

Nach dem Verfahren, das von Anker, P.; Wieland, E.; Ammann, D.; Dohner, R.; Asper, R und Simon, W. in Anal. Chem. 1981 , 53, 1970 beschrieben ist, wurde eine ionenselektive Membran hergestellt, indem man die folgenden Komponenten verwendete:

| Komponente | Gewichts-% |
|---|---|
| Verbindung der Formel Ia | 1,2 |
| Kalium-tetrakis (p-chloro-phenyl)borat | 0,4 |
| o-Nitrophenyl-octyläther | 65,5 |
| Poly(vinylchlorid) | 32,8. |

Die verwendeten 0,4 Gew.-% an Kaliumtetrakis (p-chloro-phenyl)borat entsprechen etwa 30 Mol-% an dieser Verbindung, bezogen auf die Verbindung der Formel Ia.

Beispiel 5

EMK-Messungen

Die EMK-Messungen wurden bei 20 - 22° C mit einer Vergleichselektrode und bis zu 16 ionenselektiven Elektroden gleichzeitig durchgeführt, wobei man etwa 100 ml der Probelösung verwendete. Die eingesetzte elektronische Anordnung wird von Wuthier, U.; Pham, H.V.; Zünd, R; Welti, D.; Funck, R.J.J.; Bezegh, A.; Ammann, D.; Pretsch, E.; und Simon, W. in Anal. Chem. 1984 , 56, 535 beschrieben.

Bei jeder ionenselektiven Elektrode wurde die Bestimmung der EMK alle 30 Sekunden während eines Zeitraumes von 15 - 20 Minuten durchgeführt. Der Mittelwert der letzten fünf Werte wurde zur Berechnung ausgewählt. Dieser wurde bezüglich des Flüssigkeitspotentials zwischen der Probelösung und der dreimolaren Kaliumchloridlösung des Stromschlüssels korrigiert. Die Berechnungen wurden mit der Henderson Formel nach demjenigen Verfahren durchgeführt, das von Morf, W.E. in "The Principles of Ion-Selective Electrodes and of Membrane Transport", Akadémiai Kiado: Budapest and Elsevierä Amsterdam, New Vork, 1981, beschrieben ist.

Aus den Konzentrationen wurden die Einzelionenaktivitäten nach der Theorie von Debye-Hückel erhalten. Es sei bezüglich der Gleichungen und angewandten Parameter auf die Veröffentlichungen von Meier, P.C.; Ammann, D.; Morf, W.E.; Simon W. in "Medical and Biological Applications of Electro-chemical Devices", Koryta, J., Ed.; Wiley: Chichester, New York, Brisbane, Toronto, 1980, und Meier P.C. Anal. Chim. Acta 1981 , 136, 363, verwiesen.

Die Elektrodenansprechfunktionen wurden nach der Nicolsky-Eisenman Gleichung abgeschätzt.

Die nach dem Beispiel 4 hergestellte Membran besass eine $Li^+/Na^+$-Selektivität von 280. Diese Membran wurde zur Bestimmung von Lithiumionen in Serumproben eingesetzt. Die verwendeten Serumproben enthielten 0,14 Mol Natriumchlorid, 0,004 Mol Kaliumchlorid, 0,0011 Mol $CaCl_2$ und 0,0006 Mol $MgCl_2$.

Zu den Serumproben setzte man 0,77 mMol bis 1,96 an $Li^+$ Ionen zu und die $Li^+$ Konzentrationen wurden mit einer Elektrode bestimmt, die mit der ionenselektiven Membran gemäss Beispiel 4 ausgerüstet war. Bei Verwendung von Eichkurven wurden die folgenden Lithiumkonzentrationen bestimmt:

| $c_{Li^+}$ in mMol, das zu dem Serum zugesetzt wurde | $c_{Li^+}$ in mMol, das mit 2 Sensoren bestimmt wurde (Standardabweichung) | relative Genauigkeit [%] |
|---|---|---|
| 0,77 | 0,79 (0,00) | + 3 % |
| 0,99 | 0,96 (0,02) | - 3 % |
| 1,32 | 1,31 (0,04) | - 1 % |
| 1,48 | 1,37 (0,02) | - 7 % |
| 1,96 | 1,83 (0,02) | - 7 % |

Die Nachweisgrenze log $a_{Li}$ betrug etwa -3,3 in wässrigen Lösungen, welche die im Serum vorherrschenden Konzentrationen an Alkalimetallionen und Erdalkalimetallionen enthielten.

## Ansprüche

1. Cyclohexan-cis-1,2-dicarbonsäurediamide, dadurch gekennzeichnet, dass sie die Formel I

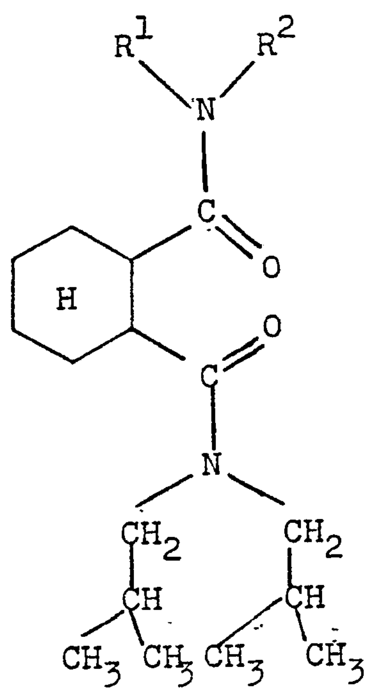

I

aufweisen, worin die Gruppierung der Formel

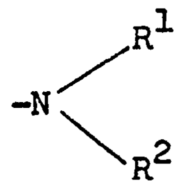

entweder die Formel

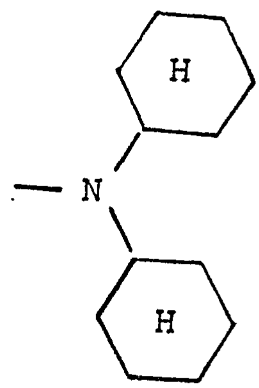

oder die Formel

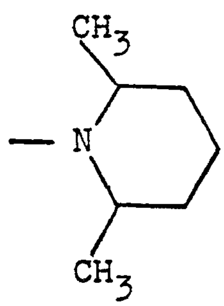

aufweist.

2. Verbindung nach Anspruch 1, dadurch gekennzeichnet, dass sie das N,N-Dicyclohexyl-N', N'-diisobutyl-cis-cyclohexan-1,2-dicarbonsäureamid der Formel Ia

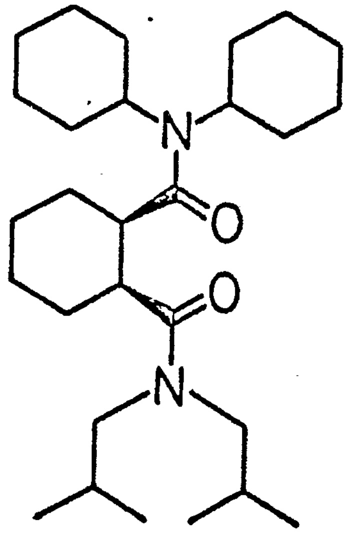

Ia

ist.

3. Ionenselektive Membran zur Bestimmung der Konzentration von Lithiumionen, dadurch gekennzeichnet, dass sie als ionenselektive Komponente eine Verbindung der Formel I gemäss Anspruch 1 enthält.

4. Ionenselektive Membran gemäss Anspruch 3, dadurch gekennzeichnet, dass sie als ionenselektive Komponente eine Verbindung der Formel Ia gemäss Anspruch 2 enthält.

5. Ionenselektive Membran gemäss Anspruch 3 oder 4, dadurch gekennzeichnet, dass sie ausserdem Poly(vinylchlorid) als Matrix für die ionenselektive Komponente enthält.

6. Ionenselektive Membran nach Anspruch 5, dadurch gekennzeichnet, dass sie Poly(vinylchlorid) als Matrix und einen Weichmacher, vorzugsweise einen lipophilen Weichmacher enthält, wobei o-Nitrophenyloctyläther oder Bis(1-butylpentyl)adipate speziell bevorzugt sind.

7. Ionenselektive Membran nach Anspruch 6, dadurch gekennzeichnet, dass sie ausserdem Tetraphenylborat und/oder Kalium-tetrakis(p-chlorophenyl) borat enthält.

8. Testvorrichtung zur Testung auf Lithiumionen in flüssigen Medien, dadurch gekennzeichnet, dass sie als Komponente eine Verbindung der Formel I gemäss Anspruch 1 enthält.

9. Testvorrichtung nach Anspruch 8, dadurch gekennzeichnet, dass sie ausserdem eine Polymermatrix und vorzugsweise einen Indikator enthält, der eine Aenderung des pH-Wertes anzeigt.

10. Testvorrichtung nach Anspruch 9, dadurch gekennzeichnet, dass sie ein Streifen oder ein Band ist, in welchem der pH-Indikator ein solcher ist, der eine Aenderung des pH-Wertes durch einen Farbumschlag anzeigt.

Ansprüche für den Vertragsstaat AT

1. Verfahren zur Herstellung von Cyclohexan-cis-1,2-dicarbonsäurediamiden der Formel I

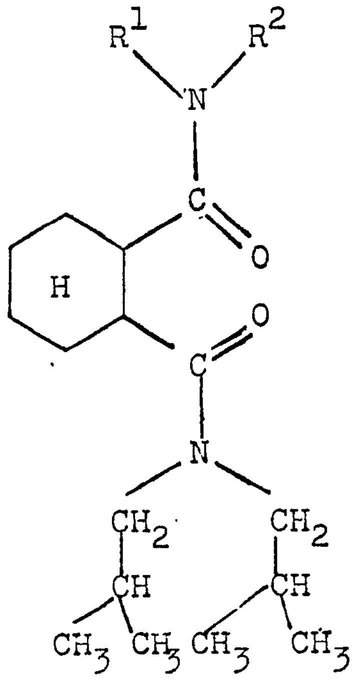

I

in welchen die Gruppierung der Formel

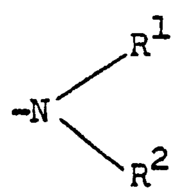

entweder die Formel

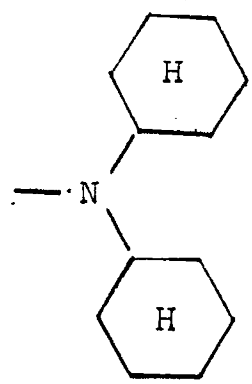

oder die Formel

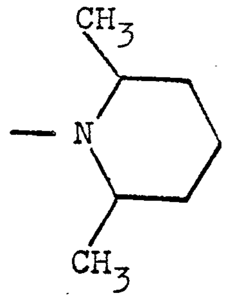

aufweist,
dadurch gekennzeichnet, dass man ein reaktives Derivat der cis-Cyclohexan-1,2-dicarbonsäure der Formel

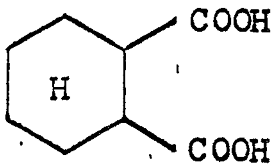

in beliebiger Reihenfolge, sowohl mit einem Amin der Formel III

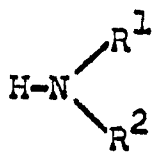

als auch mit einem Amin der Formel VI

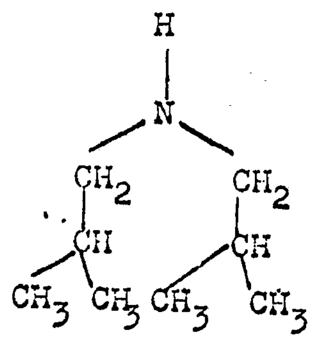

VI

zu dem Endprodukt der Formel I umsetzt.

2. Verfahren nach Anspruch 1, dadurch gekennzeichnet, dass man das N,N-Dicyclohexyl-N', H'-diisobutyl-cis-cyclohexan-1,2-dicarbonsäureamid der Formel Ia

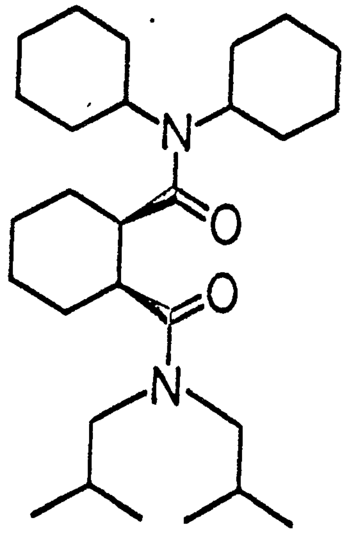

Ia

herstellt, indem man das entsprechende Cyclohexan-dicarbonsäureanhydrid in beliebiger Reihenfolge sowohl mit dem Dicyclohexylamin, als auch mit dem Diisobutylamin der Formel VI umsetzt.

**3.** Testvorrichtung zur Testung auf Lithiumionen in flüssigen Medien, dadurch gekennzeichnet, dass sie als Komponente ein Cyclohexan-cis-1,2-dicarbonsäurediamid der Formel I

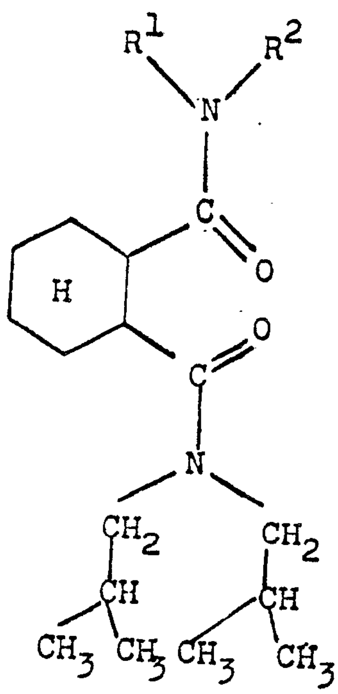

I

enthält, in welchem die Gruppierung der Formel die Formel

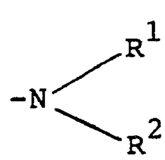

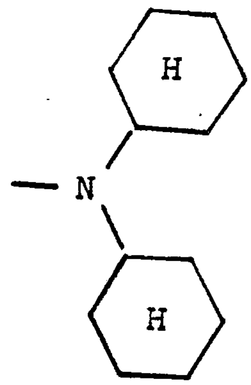

oder

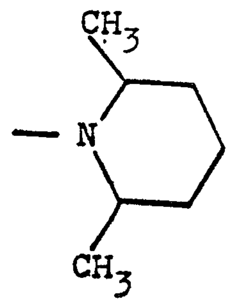

aufweist.

4. Testvorrichtung nach Anspruch 3, dadurch gekennzeichnet, dass sie ausserdem eine Polymermatrix und vorzugsweise einen Indikator enthält, der eine Aenderung des PH-Wertes anzeigt.

5. Testvorrichtung gemäss Anspruch 4, dadurch gekennzeichnet, dass sie ein Streifen oder ein Band ist, in welchem der pH-Indikator ein solcher ist, der eine Aenderung des pH-Wertes durch einen Farbumschlag anzeigt.

6. Testvorrichtung nach Anspruch 3, dadurch gekennzeichnet, dass sie eine ionenselektive Membran ist, die als Komponente ein Cyclohexan-cis-1,2-dicarbonsäurediamid der Formel I enthält.

7. Ionenselektive Membran gemäss Anspruch 6, dadurch gekennzeichnet, dass sie als ionenselektive Komponente das N,N-Dicyclohexyl-N'-N'-diisobutyl-cis-cyclohexan-1,2-dicarbonsäurediamid der Formel Ia

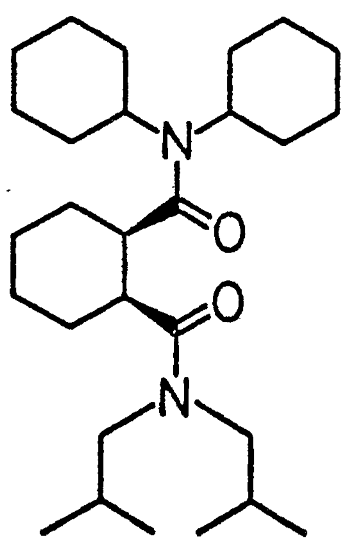

Ia

enthält.

8. Ionenselektive Membran gemäss Anspruch 6 oder 7, dadurch gekennzeichnet, dass sie ausserdem Poly(vinylchlorid) als Matrix für die ionenselektive Komponente enthält.

9. Ionenselektive Membran gemäss Anspruch 8, dadurch gekennzeichnet, dass sie Poly(vinylchlorid) als Matrix und einen Weichmacher, vorzugsweise einen lipophilen Weichmacher enthält, wobei o-Nitrophenyloctyläther oder Bis(1-butylpentyl)adipate speziell bevorzugt sind.

10. Ionenselektive Membran gemäss Anspruch 9, dadurch gekennzeichnet, dass sie ausserdem Tetraphenylborat und/oder Kalium-tetrakis(p-chlorophenyl) borat enthält.

## Claims

1. Cyclohexane-cis-1,2-dicarboxylic acid diamides, characterized in that they exhibit Formula I

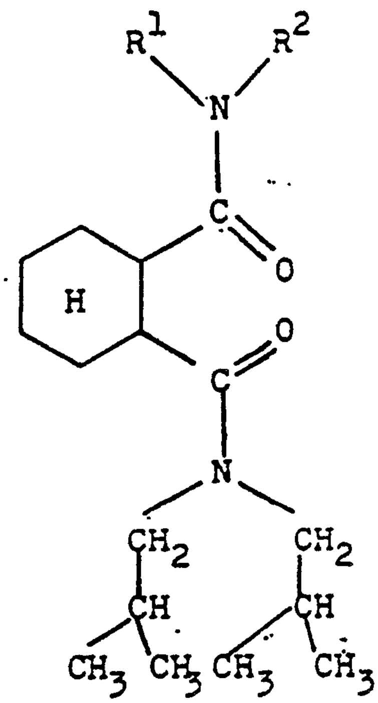

I

in which the group with the formula

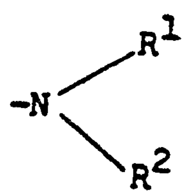

exhibits either the formula

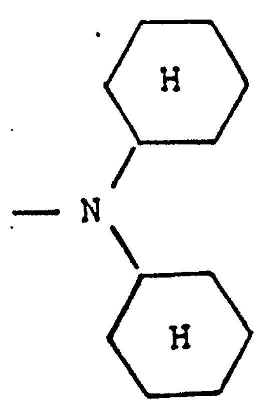

or the formula

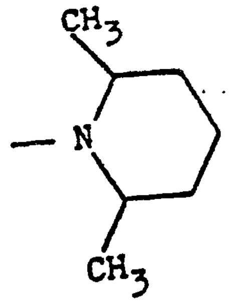

2. Compound according to claim 1, characterized in that it is the N,N-dicyclohexyl-N',N'-diisobutyl-cis-cyclohexane-1,2-dicarboxylic acid amide of Formula Ia

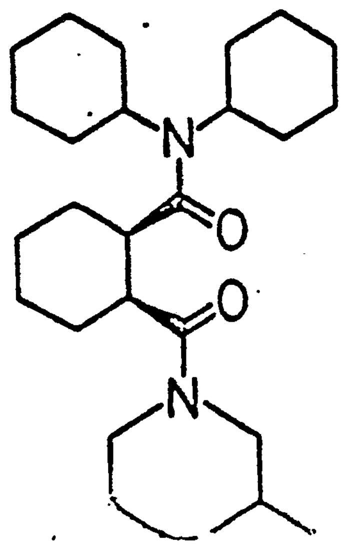

Ia

Ia

3. Ion-selective membrane for determining the concentration of lithium ions, characterized in that it contains as the ion-selective component a compound of Formula I according to claim 1.

4. Ion-selective membrane according to claim 3, characterized in that it contains as the ion-selective component a compound of Formula Ia according to claim 2.

5. Ion-selective membrane according to claim 3 or 4, characterized in that it contains in addition poly-(vinylchloride) as matrix for the ion-selective component.

6. Ion-selective membrane according to claim 5, characterized in that it contains poly(vinylchloride) as matrix and a plasticizer, preferably a lipophilic plasticizer, with particular preference being given to o-nitrophenyl-octyl ether or bis(1-butylpentyl)adipate.

7. Ion-selective membrane according to claim 6, characterized in that it contains in addition tetraphenylborate and/or potassium-tetrakis(p-chlorophenyl)borate.

8. Test device for testing for lithium ions in liquid media, characterized in that it contains as component a compound of Formula I according to claim 1.

9. Test device according to claim 8, characterized in that it contains in addition a polymer matrix and preferably an indicator which indicates a change in the pH value.

10. Test device according to claim 9, characterized in that it is a strip or tape in which the pH indicator is one which indicates a change in the pH value by a colour change.

CLAIMS for the contracting state: AT

1. Method of manufacturing cyclohexane-cis-1,2-dicarboxylic acid diamides of Formula I

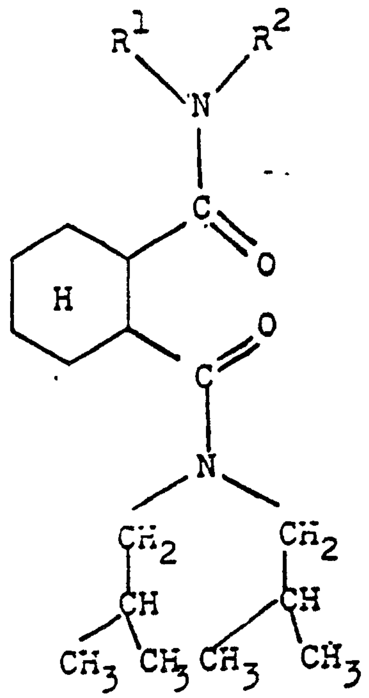

I

in which the group with the formula

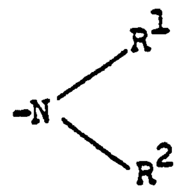

exhibits either the formula

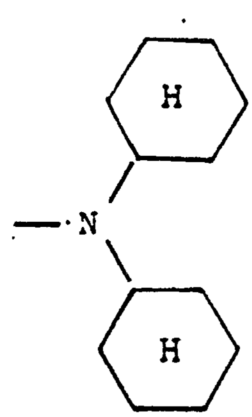

or the formula

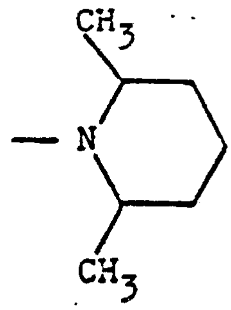

characterized in that a reactive derivate of the cis-cyclohexane-1,2-dicarboxylic acid with the formula

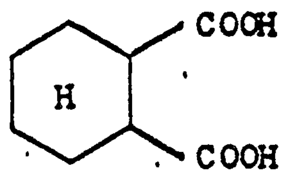

is reacted in any sequence, both with an amine of Formula III

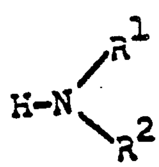

and with an amine of Formula VI

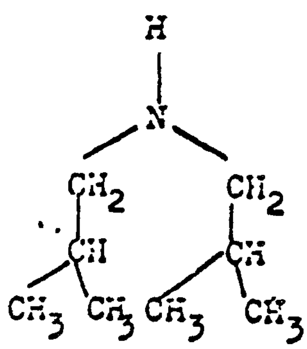

VI

to obtain the end product of Formula I.

2. Method according to claim 1, characterized in that the N,N-dicyclohexyl-N', N'-diisobutyl-cis-cyclohexane-1,2-dicarboxylic acid amide of Formula Ia

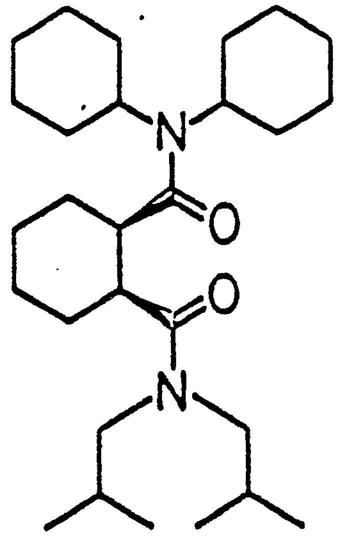

Ia

is manufactured by reacting the corresponding cyclohexanedicarboxylic acid anhydride in any sequence both with the dicyclo-hexylamine and with the diisobutyl amine of Formula VI.

3. Test device for testing for lithium ions in liquid media, characterized in that it contains as component a cyclohexane-cis-1,2-dicarboxylic acid diamide of Formula I

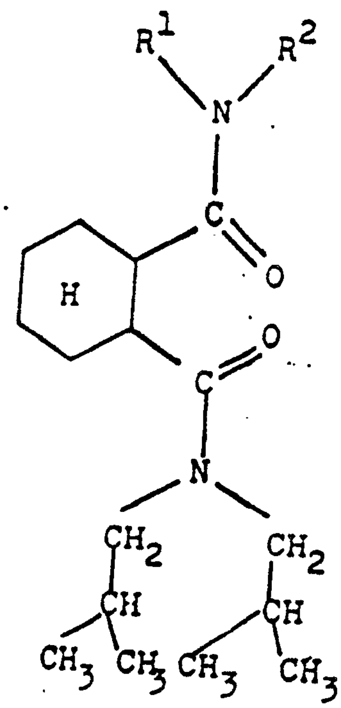

I

in which the group with the formula

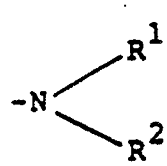

exhibits the formula

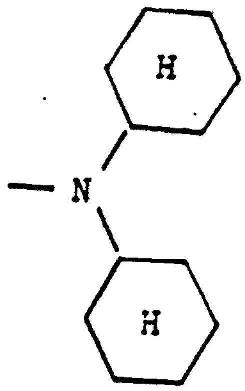

or

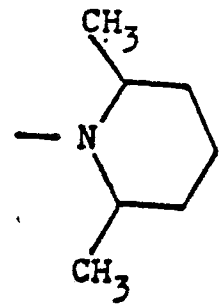

4. Test device according to claim 3, characterized in that it contains in addition a polymer matrix and preferably an indicator which indicates a change in the pH value.

5. Test device according to claim 4, characterized in that it is a strip or tape in which the pH indicator is one which indicates a change in the pH value by a colour change.

6. Test device according to claim 3, characterized in that it is an ion-selective membrane which contains as component a cyclohexane-cis-1,2-dicarboxylic diamide of Formula I.

7. Ion-selective membrane according to claim 6, characterized in that it contains as the ion-selective component the N,N-dicyclohexyl-N'-N'-diisobutyl-cis-cyclohexane-1,2-dicarboxylic acid diamide of Formula Ia

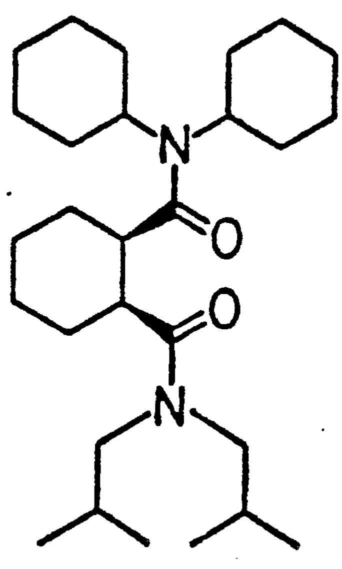

Ia

8. Ion-selective membrane according to claim 6 or 7, characterized in that it contains in addition poly-(vinylchloride) as matrix for the ion-selective component.

9. Ion-selective membrane according to claim 8, characterized in that it contains poly(vinylchloride) as matrix and a plasticizer, preferably a lipophilic plasticizer, with particular preference being given to o-nitrophenyl-octyl ether or bis(1-butylpentyl)adipate.

10. Ion-selective membrane according to claim 9, characterized in that it contains in addition tetraphenylborate and/or potassium-tetrakis(p-chlorophenyl)borate.

## Revendications

**1.** Diamides d'acide cyclohexane-cis-1,2-dicarboxylique, caractérisés en ce qu'ils répondent à la formule I

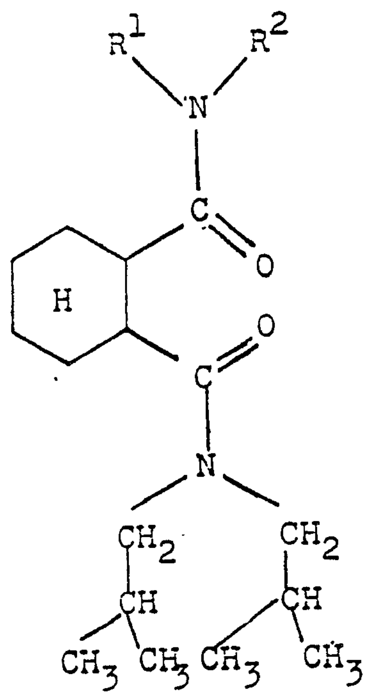

I

dans laquelle le groupement de formule

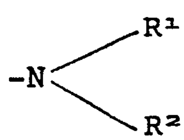

répond à la formule

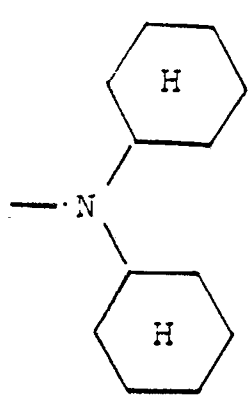

ou à la formule

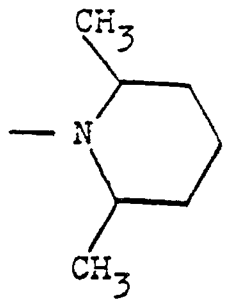

2. Composé selon la revendication 1, caractérisé en ce qu'il s'agit de l'amide d'acide N,N-dicyclohexyl-N',N'-diisobutyl-cis-cyclohexane1,2-dicarboxylique de formule Ia

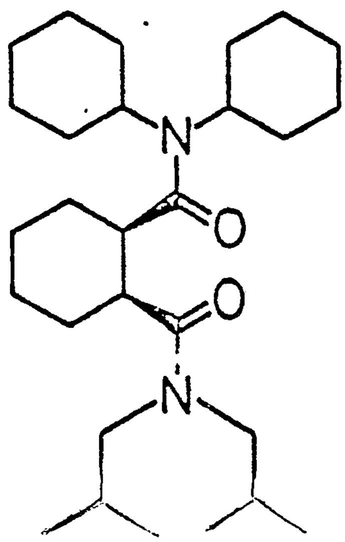

Ia

3. Membrane ion-sélective pour la détermination de la concentration d'ions lithium, caractérisée en ce qu'elle contient comme composant ion-sélectif un composé de formule I selon la revendication 1.

4. Membrane ion-sélective selon la revendication 3, caractérisée en ce qu'elle contient comme composant ion-sélectif un composé de formule Ia selon la revendication 2.

5. Membrane ion-sélective selon la revendication 3 ou 4, caractérisée en ce qu'elle contient de plus du poly(chlorure de vinyle) comme matrice pour le composant ion-sélectif.

6. Membrane ion-sélective selon la revendication 5, caractérisée en ce qu'elle contient du poly(chlorure de vinyle) comme matrice et un plastifiant, de préférence un plastifiant lipophile, l'éther o-nitrophényloctylique ou l'adipate de bis(1-butylpentyle) étant spécialement préférés.

7. Membrane ion-sélective selon la revendication 6, caractérisée en ce qu'elle contient de plus du tétraphénylborate et/ou du tétrakis(p-chlorophényl)-borate de potassium.

8. Dispositif d'essai pour le contrôle des ions lithium dans des milieux liquides, caractérisé en ce qu'il contient comme composant un composé de formule I selon la revendication 1.

9. Dispositif d'essai selon la revendication 8, caractérisé en ce qu'il contient de plus une matrice polymère et de préférence un indicateur qui indique une modification de pH.

10. Dispositif d'essai selon la revendication 9, caractérisé en ce qu'il consiste en une bande ou un ruban

dans lequel l'indicateur de pH est tel qu'une modification de pH est indiquée par un virage de couleur.

REVENDICATIONS POUR L'ETAT CONTRACTANT AT

1. Procédé pour la fabrication d'amides d'acide cyclohexane-cis-1,2-dicarboxylique de formule I

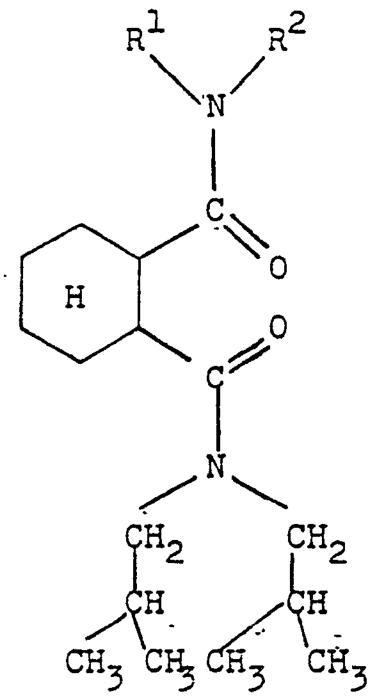

I

dans laquelle le groupement de formule

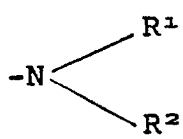

répond à la formule

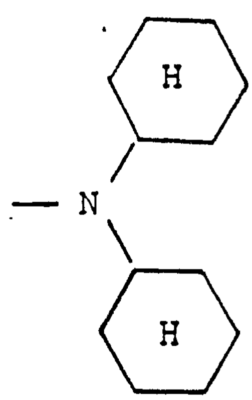

ou à la formule

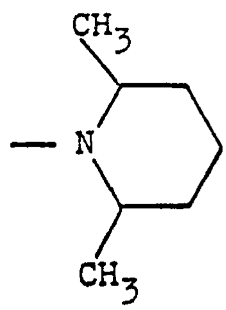

caractérisés en ce que l'on fait réagir un dérivé réactif d'acide cis-cyclohexane-1,2-dicarboxylique de formule

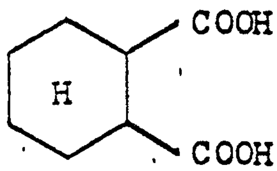

dans un ordre quelconque, tant avec une amine de formule III

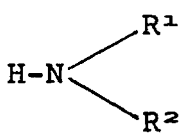

qu'avec une amine de formule VI

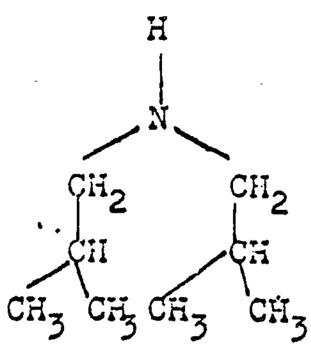

VI

et qu'on obtient le produit final de formule I.

2. Procédé selon la revendication 1, caractérisé en ce qu'on fabrique l'amide d'acide N,N-dicyclohexyl-N',N'-diisobutyl-cis-cyclohexane-1,2-dicarboxylique de formule Ia

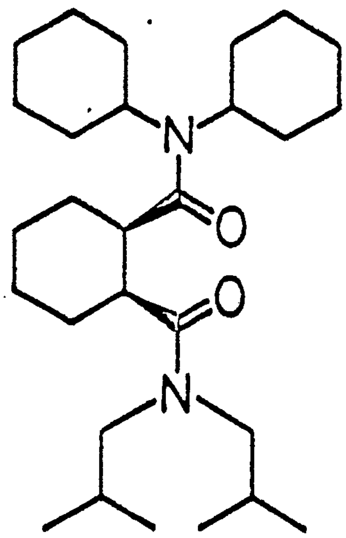

Ia

en faisant réagir l'anhydride cyclohexanedicarboxylique dans un ordre quelconque, tant avec de la dicyclohexylamine qu'avec la diisobutylamine de formule VI.

**3.** Dispositif d'essai pour le contrôle des ions lithium dans des milieux liquides, caractérisé en ce qu'il contient comme composant un amide d'acide cyclohexane-cis-1,2-dicarboxylique de formule I

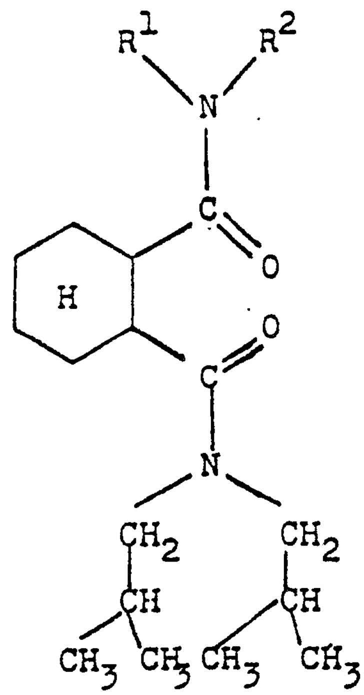

I

dans lequel le groupement de formule

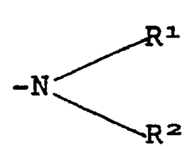

répond à la formule

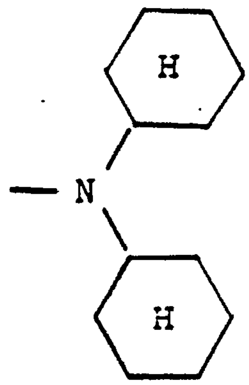

ou

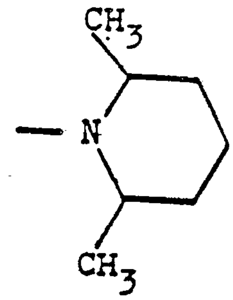

4. Dispositif d'essai selon la revendication 3, caractérisé en ce qu'il contient de plus une matrice polymère et de préférence un indicateur qui indique une modification de pH.

5. Dispositif d'essai selon la revendication 4, caractérisé en ce qu'il consiste en une bande ou un ruban dans lequel l'indicateur de pH est tel qu'une modification de pH est indiquée par un virage de couleur.

6. Dispositif d'essai selon la revendication 3, caractérisé en ce qu'il consiste en une membrane ion-sélective qui contient comme composant un diamide d'acide cyclohexane-cis-1,2-dicarboxylique de formule I.

7. Membrane ion-sélective selon la revendication 6, caractérisée en ce qu'elle contient comme composant ion-sélectif le diamide d'acide N,N-dicyclohexyl-N'-N'diisobutyl-cis-cyclohexane-1,2-dicarboxylique de formule Ia

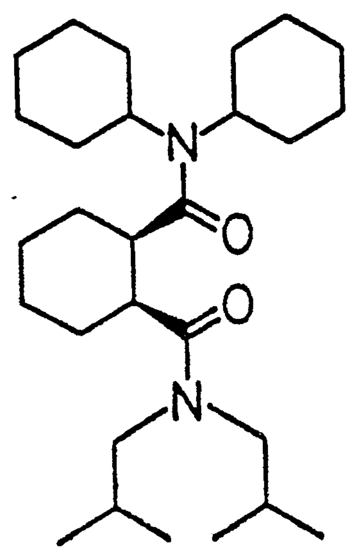

Ia

8. Membrane ion-sélective selon la revendication 6 ou 7, caractérisée en ce qu'elle contient de plus du poly(chlorure de vinyle) comme matrice pour le composant ion-sélectif.

9. Membrane ion-sélective selon la revendication 8, caractérisée en ce qu'elle contient du poly(chlorure de vinyle) comme matrice et un plastifiant, de préférence un plastifiant lipophile, l'éther o-nitrophényloctylique ou l'adipate de bis(1-butylpentyle) étant spécialement préférés.

10. Membrane ion-sélective selon la revendication 9, caractérisée en ce qu'elle contient de plus du borate de tétraphénylborate et/ou du tétrakis(p-chlorophényl)-borate de potassium.